# EUROPEAN PATENT APPLICATION

(11) **EP 1 712 343 A1**
(43) Date of publication of application: **18.10.2006**
(21) Application number: 05075857.2
(22) Date of filing: 12.04.2005
(51) Int. Cl.: B29C 67/00, A61L 27/46, A61L 27/44

(54) **Method of producing a composite article by Solid Freefrom manufacturing**

(71) Applicant: Stichting Dutch Polymer Institute, 5612 AB Eindhoven (NL)
(72) Inventor: Van Ee, Renz Jeroen, 3991 XV Houten (NL); Fischer Sabine, 5731 TP Mierlo (NL)
(74) Representative: Winckels, Johannes Hubertus F.

(57) **Abstract**

The present invention relates to a method for manufacturing a composite article, comprising
- providing a fluid mixture comprising (i) a polymeric matrix material and (ii) a dissolved inorganic component;
- forming the fluid mixture into a desired shape by Rapid Manufacturing (RM);
- allowing the matrix to solidify;
- and allowing the dissolved inorganic components to form solid particles in the matrix material.

## Description

The invention relates to a method for manufacturing a shaped composite article by a rapid manufacturing technique.

Rapid manufacturing (also referred to in the art as rapid prototyping, 3D modelling, and the like) forms a well known group of techniques that allows accurate, highly reproducible production of three-dimensional articles, without needing a mould. In particular, Rapid manufacturing (RM) can be executed via different rapid prototyping techniques (RPT). In RPT three dimensional components are directly built, layer-by-layer, from computer data description as files of 3-D models (computer aided design, CAD). The fabrication process starts with the creation of a CAD file of the 3D part followed by mechanical slicing of the CAD model into several (thin) layers. Such data description is then used to build the part in a layer wise fashion without using moulds, dyes or hard tooling. The build up of the 3D articles may be realised using different physical principles:
1. using solid materials: Extrusion of wire-like solid materials including melting and solidification: Fused deposition modelling (FDM), 3Dprinting/plotting of solid particles glued together by a binder (3D printing) or sintering of layers formed of powders of a solid by a laser beam (selective laser sintering -SLS)
2. using solid sheet-like materials: cutting and connecting of cut parts (Layered laminated manufacturing -LLM) and cutting and polymerisation (solid-foil polymerisation - SFP)
3. using fluid materials: polymerisation by heat (thermal polymerisation - TP), polymerisation by means of light interference (beam interference solidification - BIS) and polymerisation by means of one laser beam (stereolithography-SL) and by evaporation of a solvent (ink-jet printing).
4. using two fluids which form a solid component when coming into contact with each other: by 3D plotting techniques or by precise extrusion manufacturing (PEM) due to precipitation of the dissolved solids (polymer etc.) either by coagulation or by reaction.

Rapid manufacturing may be used to make composite articles comprising a polymeric matrix and solid particles embedded therein. It is a challenge to manufacture articles with improved properties such as better mechanical properties and/or better bioresorbility.

In known processes it is essential that matrix and particles are mixed very thoroughly to obtain a satisfactory distribution of the particles throughout the matrix. A bad distribution and/or agglomeration of particles easily leads to a final product with poor properties.

One may modify the particles to improve compatibility with the matrix material. However, such modification is generally complicated, and often aggregation cannot be fully avoided.

T.H. Ang et al. [Materials Science and Engineering C 20 (2002) p35-42] describe the manufacture of composite scaffolds for tissue engineering. In the described process, chitosan (as a matrix material) is dissolved in diluted acetic acid (2 % v/v) to form a hydrogel. In an embodiment hydroxyapatite (HA) microparticles, having a size of about 3-10 µm, are mixed with the solution, thereby forming a dispersion of the particles in the gel, wherein the particles remain essentially undissolved. The hydrogel is then extruded into a bath mixture of sodium hydroxide solution and ethanol, followed by a 5 min. immersion in ethanol. After washing with water the scaffolds are rapidly frozen.

It has been found by the present inventors that this method leads to an article wherein the hydroxyapatite particles form agglomerates, which may be detrimental to the quality of the article. In particular, it has been found by the inventors that by mixing the matrix material with inorganic particles such as hydroxyapatite particles (for instance nanoparticles) prior to the rapid manufacturing, a fluid mixture is obtained with a low homogeneity of the particles and/or a high tendency of the particles to agglomerate. As a result, the distribution of the particles in the shaped article has been found poorly controllable.

It is an object of the present invention, to provide a method which improves the quality/performance of a composite article obtained by a RM technique.

In particular, it is an object of the invention to provide a method which allows the manufacture of a shaped composite article by a rapid manufacturing technique whereby the preparation of the composite material is simplified and/or improved, compared to a prior art technique such as described by Ang *et al.*, which method allows a good control of the particle distribution in the article, and/or which method allows the manufacture of an article with an improved property, such as improved bioresorbility (in case of an implant), an improved mechanical property, improved processability and/or easier processability.

More in particular, it is an object of the invention to provide such a method which allows the manufacture of a shaped composite article, comprising inorganic nanoparticles.

It is further an object of the invention to provide a novel composite article, obtainable by a method according to the invention.

The inventors have now found that it is possible to produce a composite article, comprising a matrix material and particles (comprising inorganic components) in the matrix material, wherein the particles are formed *in situ, i.e.* upon and/or after forming the shaped article by the rapid manufacturing technique.

Accordingly, the present invention relates to a method for manufacturing a composite article, comprising
- providing a fluid mixture comprising (i) a polymeric matrix material and (ii) at least one dissolved inorganic component (said inorganic component being capable of forming particles in the matrix material, alone and/or in combination with one or more other inorganic components);
- forming the fluid mixture into a desired shape by Rapid Manufacturing (RM);
- allowing the matrix to solidify;
- and allowing the dissolved inorganic component or components to form solid particles in the matrix material.

*Figure 1 as a schematic drawing of a printing pattern (left) and of the electro-pneumatic apparatus for the RM (3D plotting) of a scaffold (right).*
*Figure 2 shows two different examples of printing patterns (left) and the scaffolds (right) prepared while moving the bank of a Roland DG with 20 mm*/*s in x and y direction following this pattern. By moving the injection unit into z-direction a 3-dimensional sample were built up to 20 layers.*
*Figure 3 shows a wet scaffold prepared out of a mixture of chitosan, acetic acid, NaH*_{*2*}*PO*_{*4*} *and CaCl*_{*2*}*-(Processing mixture 2b, chitosan: HA ration 50 : 50 w* / *w %) injected into a 0.1 M NaOH solution*
*Figure 4 shows an X-ray graph (XRD) of a dry conzposite sample (prepared with mixture 1 = fig. 4α and prepared with mixture 2α + fig. 4b), compared to the pure components (pure HA powder, chitosan, powder, calcium hydroxide powder). The peaks (at 26°, 32° and 40°) of the composite materials clearly proof the formation of hydroxyapatite.*
*Figure 5 illustrates the formation of HA depending on the period of time the scaffold was immersed into the coagulation bath (0.1 M NaOH) for the system*
   *a) chitosan*/*NaH2PO*_{*4*} *solution with Ca(NO*_{*3*}*)*₂= *processing mixture 2α (4 graphs on the bottom) and*
   *b) chitosan*/*NαH*_{*2*}*PO*_{*4*} *solution with CaCl*_{*2*} *=processing mixture 2b (2 graphs on the top)*
   *with α calculated chitosan: HA ratio of 60: 40 w*/*w%.*
*Figure 6 shows scanning electron microscope (ESEM) pictographs of the surface (Fig. 6a and 6 b) and of the inner part inspected on the surface of* α *broken sample (fig. 6c) of a dried scaffold prepared out of processing mixture 2b (chitosan: HA ratio 50: 50 wlw%) injected into a 0.1 M NaOH solution.*
*Figure 7 shows a thermo-grauimetrical analysis (TGA) measurement of a dried scaffold prepared fromsample composed of chitosan and hydroxyapatite processing mixture 2b. The calculated chitosan : HA ratio was 30 : 70 w*/*w% and the measured ratio by TGA was 45.2 : 54.8* (top curve: weight %; bottom curve 1^{st} derivative of weight curve).

A method according to the invention may very suitably be used to produce an article selected from the group consisting of medical devices, in particular implants, such as bone implants, teeth implants or drug delivery devices; foams; membranes; master models for precisions casting and prototypes of an article.

The method according to the invention has been found advantageous from a processing point of view. As the forming of the particles takes place *in situ* during/after shaping the article inside the (fluid mixture comprising the) polymeric matrix, the process is simplified/improved, compared to conventional processes wherein first the particles of the inorganic components are made, then the particles are mixed with the matrix material, and only thereafter the mixture is shaped.

In addition, it has been found that in a method of the invention, a composite article is formed wherein the particles of the inorganic components do not form clusters or aggregates, or at least are less aggregated than with a comparable process wherein the particles are dispersed in a solution of the matrix material, and thereafter subjected to the shaping.

As a result, a method according to the invention is very well controllable with respect to the particle distribution in the produced articles. Thus, the invention provides a way to manufacture articles with a high level of reproducibility not only with respect to the general shape and size of the article, but also with respect to particle distribution in the article.

Moreover, it has been found that a method according to the invention, is in particular suitable to produce a composite article wherein the inorganic component or components form nanoparticles, *i.e.* particles wherein at least one of the dimensions of the particles (such as length, width, thickness; or in case of an essentially spherical: particle diameter), as determined by electron microscopy is 1000 nm or less, in particular in the range of 1-500 nm. In a particular embodiment, the largest dimension of the nanoparticles is in the range of 1-1000 nm, more in particular in the range of 1-500 nm.

The particle size may further be influenced by nucleation effects, number of nuclei present, number of complexing sites provided by the matrix, precipitation kinetics, diffusion coefficients and other effects.

In accordance with the invention, it is possible to manufacture a nanocomposite article, showing an advantageous particles distribution in the article.

It has been found that the method of the invention can be used to produce a shaped article with a good dimensional stability.

It has been found that the layers of the matrix material comprising the inorganic components which may be formed during the RM adhere well in a method according to the invention.

Moreover, it has been found possible to produce an article with a highly homogenous distribution of the particles in the matrix material.

It is also possible to provide an article wherein the particles are distributed in the article in a gradient manner, *i.e.* an article wherein the concentration of particles (the number of particles per volume unit) varies from surface towards the centre of the article. In an embodiment, the volume fraction of the particles is higher at and near the surface than in the middle of the article. In an embodiment, the centre of the article is essentially free of particles.

The matrix material is present in the fluid mixture (dissolved or dispersed) and serves to maintain the desired shape of the article after the RM shaping (such as by 3Dprinting/plotting/precise extrusion) of the fluid mixture.

As a matrix material, in principle any material may be used that can be made fluid, such as by melting or by dissolving. Particularly suitable are thermoplastic polymers and polymers that can be dissolved in water or an aqueous liquid. Preferably the matrix material comprises at least one compound selected from the group consisting of polysaccharides, (poly)peptides and polyesters and mixtures thereof. Preferred are polysaccharides, including heteropolysaccharides (i.e. polysaccharides (also) comprising moieties other than -OH and ―H attached to carbon atoms of the polysaccharide).

More preferably, the matrix material comprises at least one polysaccharide selected from the group consisting of starch, carrageenan, agar, carboxylated polysaccharides such as alginates, pectins, and xanthans, and polysaccharides comprising amine groups, such as chitosan.

Polysaccharides and/or other materials comprising polar groups or sites of polarity, are in particular considered advantageous for producing an article comprising a salt as the solid particles. Without being bound by theory, it is thought that matrix polymers comprising polar groups, such as hydroxyl groups, carboxylic acid groups, amine groups and/or other groups having a similar or higher polarity than any of these, may serve as an anchor point or nucleation site for the formation of the particles.

It is further found that the presence of polar groups and/or sites may contribute to the final shape of the particles comprising inorganic components. It may for instance be useful to promote the formation of elongate (e.g. needle-like) particles.

Very good results have been achieved with a matrix material comprising -NH₂ groups, such as chitosan or another polysaccharide or a (poly)peptide or polyester comprising such groups.

It is also possible to use a matrix material without specific polar sites or groups.

The formation and shaping of the solidified nanoparticles may be governed by diffusion processes, temperature gradients and changes and micro-voids formed due to rapid coagulation of the matrix.

At least one inorganic component serves as precursor for the *in-situ* synthesis of the particles. In an embodiment all inorganic components are present (dissolved) in the fluid mixture prior to the shaping by RM. In another embodiment, at least one of the inorganic components is allowed to migrate into the shaped article, in particular by letting that component diffuse into the shaped article. This may be started while the matrix material is still solidifying and/or thereafter.

As an inorganic component, in principle any component can be used that is capable of (precipitating, and thereby) forming particles in the matrix material (at least in combination with one or more other components, that are present in the fluid mixture, or are allowed to migrate into the shaped article).

Particularly suitable inorganic components include components selected from the group consisting of inorganic cations and inorganic anions (salts, minerals). Chemicals which - in contact with the fluid in which de matrix material is dissolved form stable phases as precipitates like oxides, oxyhydrates or the like may also be used.

Particularly suitable inorganic cations include metal ions such as alkali metal ions, alkaline earth metal ions and transitional metal ions. Preferred inorganic cations include calcium, magnesium and iron ions.

Particularly suitable inorganic anions include halogen anions (such as fluoride, chloride, bromide, iodide), sulphide and anions comprising oxygen. Preferred anions include carbonate, sulphide, phosphate and hydroxide.

Usually at least one inorganic anion and at least one inorganic cation are used to form the particles. Optionally one or more organic particle forming components may be used in combination with the inorganic components, for example at least one component selected from organic ions and biologically active components such as growth hormones , medicaments and the like (in particular in case of an implant).

The particles may be crystalline, amorphous or a combination thereof.

The invention has been found very suitable to form composites comprising solid mineral particles, in particular particles selected from the group consisting of particles comprising at least one material selected from phosphates, such as hydroxyapatite; carbonates, such as hydrotalcite; metal oxides, including mixed oxides (oxides comprising cations of at least two different metals); oxyhydrates and mixed hydroxides such as layered double hydroxides (LDH).

This may be realised by dissolving the mineral/salt itself or by dissolving salts that - when precipitated together - form particles comprising the mineral/salt.

In particular for producing a composite comprising hydroxyapatite, one may dissolve a sufficiently soluble calcium salt such as Ca(NO₃)₂ , CaCl₂ or another salt having a similar or better solubility in the mixture (usually an aqueous solution) and a sufficiently soluble phosphate salt such as NaH₂PO₄ or another salt having a similar or better solubility in the mixture.

In an embodiment parts of the ions needed for the formation of the (HA) particles (such as phosphates) are dissolved in the mixture and other parts (such as calcium and hydroxide ions) are allowed to migrate from the coagulation bath into the shaped article or the other way around.

A fluid mixture which is processed during the shaping procedure is considered fluid, if it is flowable when shaping the article by RM. Thus, the mixture is at least made to flow (e.g. by using pressure) by the equipment used in the shaping by rapid manufacturing. The viscosity is not particularly relevant, as long as it can be made to flow by the used equipment. The skilled person will know how to select conditions such that the fluid is made to flow by the RM equipment.

The fluid mixture may be highly viscous, as long as the viscosity is such that it can be processed during shaping by the RM equipment.

The fluid mixture may be a solution wherein both the matrix material and the inorganic components have been dissolved. A suitable solvent depends on the nature of the matrix material and nature of the inorganic components. Solvents which may be used include solvents comprising water, alcohols, ethers, esters, acids, including mixtures thereof. Good results have been achieved with water or an aqueous solution as a solvent.

The skilled person will know how to select a suitable solvent based upon the properties of the matrix material and the inorganic components.

In addition to the matrix material, inorganic components and optionally the solvent, one or more additives may be present in the fluid mixture. In particular, the mixture may comprise one or more components selected from the group consisting of pH-modulators (acids, bases), Where the inorganic components form a salt, an acid of which the acid- anion is the same as (one of) the anion(s) of the salt forming the solid particles may be used as an acid modulator. A hydroxide of which the cation is the same as (one of) the cation(s) of the salt forming particles, may be used as a base modulator.

Preferably the amount of the matrix material in the fluid mixture is in the range of 20 to 99 wt. % based on the total weight the matrix material plus inorganic components, more preferably in the range of 50 to 90 wt. % based on the total weight the matrix material plus inorganic components.

The amount of the particle forming (inorganic) components is preferably in the range of 1 to 80 wt. %, based on the total weight of the matrix material plus inorganic components, more preferably in the range of 10 to 50 wt. %.

If present, the amount of solvent is preferably 99 wt. % or less, more preferably in the range of 50 to 99 wt. %, based on the total weight of the fluid mixture.

In particular, Rapid Manufacturing (RM) can be executed via one or more of different rapid prototyping techniques (RPT). In RPT, three dimensional components are directly built, layer-by-layer, from computer data description as files of 3-D models (computer aided design, CAD) The fabrication process start with the creation of a CAD file of the 3D part followed by mechanical slicing of the CAD model into several (thin) layers. Such data description are then used to build the part in a layer wise fashion without needing moulds, dies or hard tooling. The build up of the 3D articles may be realised using the following physical principle: Using two fluids which form a solid article when coming with each other into contact: by 3D plotting/printing techniques or by precise extrusion manufacturing -PEM due to precipitation of the dissolved solids (polymer etc.) either by coagulation or by reaction.

Suitable equipment is known in the art. For instance the 3D plotting technique, e.g. as described by T.H. Ang et al. [Materials Science and Engineering C 20 (2002) p35-42] is suitable, with the proviso that in the present invention the inorganic components are formed *in-situ* during the processing instead of dispersed in advance as solid particles.

The forming into a desired shape (shaping), solidifying and/or particle formation may be carried out subsequently or (at least partially) simultaneously, for instance all in a single liquid bath (see also below). For practical reasons, the solidifying usually starts during the shaping and may continue after the shaping is completed. The formation of the particles may start during and/or after shaping. The formation of the particles may start during and/or after solidifying.

Generally, the formation of the particles is continued after shaping. For practical reasons, the formation of the particles is usually continued after the matrix material has solidified. In practice, the invention allows to continue the particle formation after solidification of the matrix material, without needing to change a process parameter, for instance changing the liquid in the bath or changing the bath itself (although this may be done if desired). Thus, even when the shaping (such as by 3Dprinting/plotting of the mixture), solidifying and particle formation are time-wise (partially) separated, the process is simplified compared to a process wherein first the particles are made, then the particles are mixed with the matrix material (usually a complicated and time consuming process step) and thereafter the mixture is shaped.

The shaping by RM is usually carried out in a liquid bath, wherein the article is submerged as it is being formed. The liquid may be a liquid in which the matrix material solidifies (see also below).

The solidification of the fluid mixture may be carried out in any way in which the matrix material can be solidified. The matrix material is considered solidified within the context of the invention when the shaped material is essentially shape stable when being positioned on a flat surface, under processing conditions (temperature, pressure, the medium surrounding the material and the like).

The solidification of the matrix material may involve formation of a crystalline solid matrix phase, of a rigid gel structure, of an amorphous solid matrix phase.

A very suitable way to solidify the matrix material is to expose it to a liquid wherein the material (largely) does not dissolve ( a coagulation medium). Such exposure is preferably carried out in a coagulation bath wherein the shaped fluid mixture is submerged. More preferably, the shaping with the rapid manufacturing is carried out in the coagulation bath.

Alternatively, the matrix material may be contacted with coagulation medium by spraying or the like.

Such liquid may be a non-solvent for the matrix material, such as a hydrophilic solvent/dispersing medium or water for a hydrophobic matrix material or a hydrophobic solvent/dispersing medium for a hydrophilic matrix material. For instance, ethanol or a liquid of similar or more hydrophobicity may be used to solidify a polysaccharide or a matrix material having a similar or higher hydrophilicity (usually by precipitation out of an aqueous medium forming part of the fluid mixture).

Another way to effect solidification is to use a liquid having a pH at which the matrix material (largely) does not dissolve, *i.e.* a liquid having a high pH in case the matrix material only dissolves in the liquid at a low pH or a liquid having a low pH in case the matrix material only dissolves in the liquid at a high pH. For instance, a matrix material comprising groups that may form cations (at low pH) - such as polymers comprising amine groups, for instance chitosan - may dissolve well at low pH but poorly or not at all at a sufficiently high pH.

Yet another way to effect solidification is to expose the fluid mixture to a liquid of high ionic strength.

The formation of the particles comprising the inorganic components may be effected in any way suitable for achieving the particle formation of said components. Suitably ways, include those described above for the solidification of the matrix material. Particularly suitable are the use of a liquid which is a non-solvent and/or which has a pH at which the inorganic components (in combination) do not (fully) dissolve (at the concentration in which the inorganic components are present).

In a preferred embodiment, the matrix material and inorganic components are chosen such that a condition exists at which they both solidify respectively form particles, for instance they are chosen such that a suitable non-solvent exists. Preferably they are chosen such that they both dissolve at a relatively high pH or a relatively low pH, yet they solidify respectively form solid particles at a relatively low pH respectively a relatively high pH. For instance, polysaccharides comprising amine groups, such as chitosan dissolve well in concentrated acidic solutions and so do many salts, but may be solidified respectively precipitated (thereby forming the particles) by exposing it to an alkaline liquid. A suitable alkaline liquid is a hydroxide solution, preferably an aqueous hydroxide solution, more preferably an aqueous hydroxide solution comprising at least 0.001 M of at least one hydroxide dissolved therein even more preferably an aqueous hydroxide solution comprising 0.05 to 3 M of at least one hydroxide dissolved therein. Particularly suitable hydroxides include NaOH, KOH, Ca(OH)₂ and Mg(OH)₂.

An advantage of choosing conditions wherein both the matrix material solidifies and the inorganic components form particles is that both solidification and particle formation can be carried (at least partially) simultaneously or at least within a (partially) combined processing step. In particular solidification of the matrix material and particle formation of the inorganic components may then be carried using a single coagulation medium (usually in a coagulation bath).

In practice, it has been found that the solidification of the matrix material is advantageously realised within a relatively short time span, in particular within 120 min, preferably within 3 sec to 30 min, whereas the particle formation may be proceeded for a longer time, in particular for at least about 0.5 hour, counted from the start of the exposure to the coagulation medium.

It has been found that by selecting the time period during which the mixture comprising the matrix material and the inorganic components are exposed to the coagulation medium and thus allowing the components for the formation of the particles to migrate from the coagulation bath into the article), it is possible to control the size, number and/or shape of the particles. Besides it has been found that the distribution of the particles through the matrix material can be controlled by selecting the exposure time to the coagulation medium. In particular it has been found, that it is possible to provide an article wherein more particles comprising inorganic components is found near the surface of the article than near the core. This may be achieved by carrying out the particle formation for a relatively short time, in particular less than about 2 hours, more in particular less than about 0.5 hour. Based on the information described present application, it will be understood by the skilled person that the concentration of components in the liquid bath (coagulation bath) may be altered in order to influence the speed of the particle formation.

In particular, for a highly uniform distribution throughout the article, it is preferred to carry out the particle formation for a relatively long time, in particular at least 3 hours, more in particular at least 8 hours even more in particular at least 24 hours.

In principle the upper time limit for particle formation is not critical. For practical reasons (time-related), the particle formation is usually carried out for up to about 48 hours in particular for 24 hours or less, more in particular for 12 hours or less.

After particle formation, the article may be used immediately or further processed, for instance washed with demineralised water and/or dried.

Depending upon the intended use, one or more specific further steps may be employed. For instance, in case of an implant or a delivery device for drugs it may first be sterilised. In case the article is to be used as an implant it may be seeded with precursor cells that may proliferate and differentiate into the type of cells for the tissue that the implant is supposed to replace. Thus the articles may be seeded with bone precursor cells, in case of a bone implant.

The invention further relates to a shaped article, obtainable by a method according to the invention.

The article may have a homogenous distribution of the particles throughout the material.

An article according to the invention may be isotropic or anisotropic. Such an article may for instance be an implant, in particular a bone implant, a tooth implant or the like. In case of a bone implant, the implant preferably comprises calcium phosphate particles as the solid particles, in particular hydroxyapatite particles.

The invention further relates to an implant, wherein the shaped article according to the invention is seeded with cells, in particular precursor cells for bone, teeth or another tissue.

The invention will now be illustrated by the following example.

### Example

### Materials and Methods

Chitosan (high molecular weight), phosphoric and acetic acid, calcium nitrate and chloride as well as sodium and calcium hydroxide were obtained from Aldrich Chemical Co.

Chitosan was dissolved in aqueous 1 w% acetic acid solution in a 3 wt.% concentration by stirring at 40°C overnight (called below: chitosan solution).

### Preparation processing mixture 1:

The chitosan solution was mixed with a 8.5 w% aqueous solution of phosphoric acid (ratio 32,74 : 67,26 w%) by stirring for an hour. The solution was allowed to nature at room temperature overnight for ageing.

### Preparation processing mixture 2a:

An amount of 0.50g of Ca(NO₃)₂ was added to 31.40g of the chitosan solution. The mixture was heated in a water bath to 60°C and stirred until all Ca(NO₃)₂ was dissolved. Subsequently 0.17g of NaH₂PO₄ was added to this mixture, heated in a water bath to 60°C and stirred until all NaH₂PO₄ was dissolved. The calculated wt/wt% ratio of chitosan : HA (which can be formed out of the added Ca(NO₃)₂ and NaH₂PO₄) was 60/40. The mixture was used directly for the 3D plotting process to avoid a precipitation of calcium phosphates.

### Preparation processing mixture 2b:

The mixture 2b was prepared in the same way like the mixture 2a described above, replacing Ca(NO₃)₂ by CaCl₂ using the amounts listed in the table below to prepare mixtures with different calculated chitosan : HA ratios.

**Table 1: Used quantities of components of processing mixture 2b to achieve different rations of chitosan and HA in the final product**

| **Chitosan/HA** (wt/wt%) | **Chitosan** (g) | **CaCl**_{**2**} (g) | **NaH**_{**2**}**PO**_{**4**} (g) |
|---|---|---|---|
| 60/40 | 0.66 | 0.28 | 0.17 |
| 50/50 | 0.86 | 0.55 | 0.34 |
| 40/60 | 0.73 | 0.64 | 0.43 |
| 30/70 | 0.58 | 0.82 | 0.52 |

The RM process was performed by using an electro-pneumatic dossier unit Techcon System TS9701 from Unitek EAPRO suitable for high viscose liquids with a 10cc and 30cc dossier unit steered by a computer aided modelling machine Camm-3, Roland DG model PNC-3000 (software van Roland, drEngrave version 1.90).

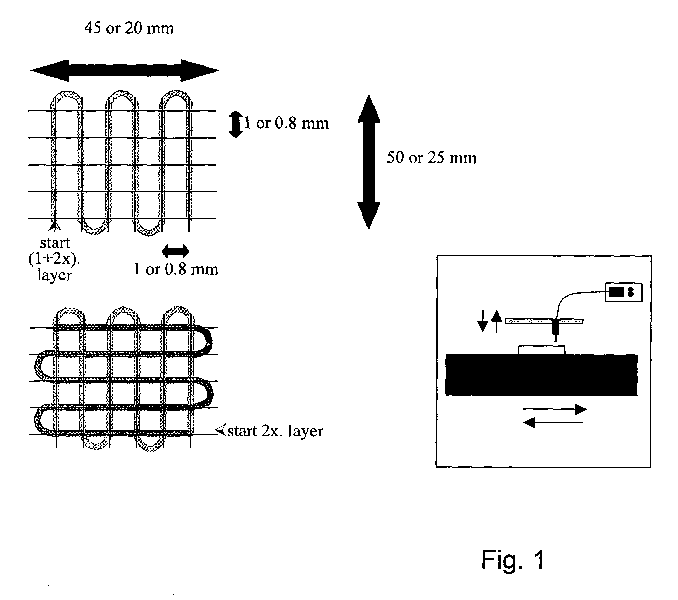

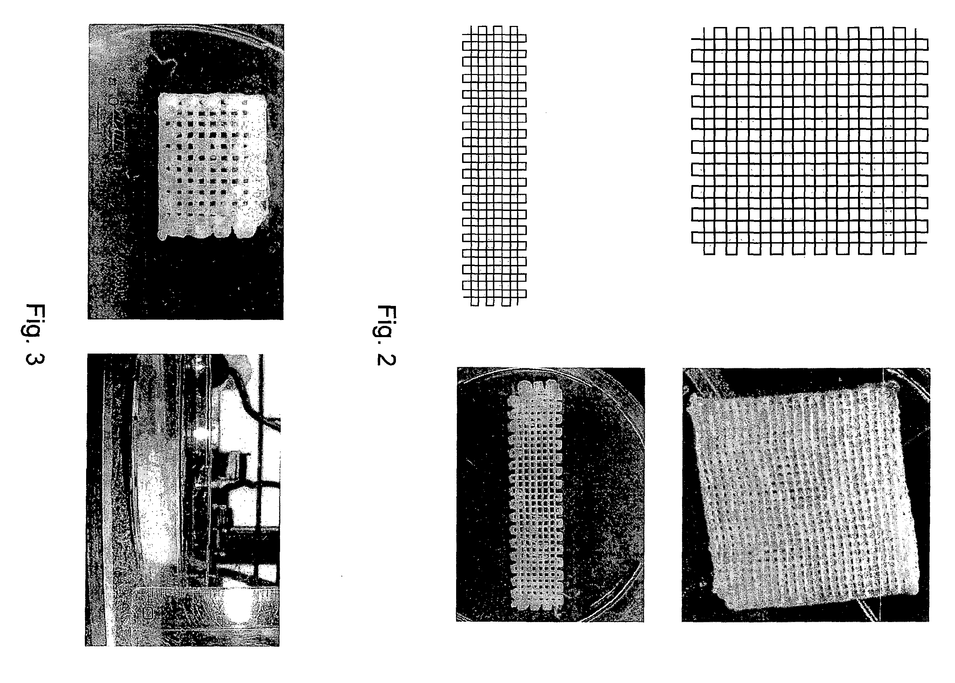

The starting mixtures were forced trough the injection nozzle by applying air pressure (1,5 bar) and injected into a coagulation bath (0.5 M Ca(OH)₂ aq. solution for mixture 1 and 0.1M NaOH aq. solution for mixtures 2a and 2b) at room temperature via the electro-pneumatic dossier unit (see fig. 1 right side). The processing mixtures leaved the injection nozzle and solidified in the alkali coagulation bath by a pH reaction of chitosan. While moving the bank of the Roland DG with 20 mm/s in x and y direction a 2-dimensional pattern was prepared (see fig. 1 left side and figure 2). By moving the injection unit into z-direction a 3-dimensional sample was built up to 20 layers (see figure 3).

The analysis of the morphology and homogeneity of the samples were done by using a Philips ESEM (1 kV, high vacuum, spot size 3) the composition of the samples were analysed by using XRD Technique. The relation of inorganic : organic components of the final composite was determined by TGA (Perkin Elmer).

### Results:

### RM processing with mixture 1

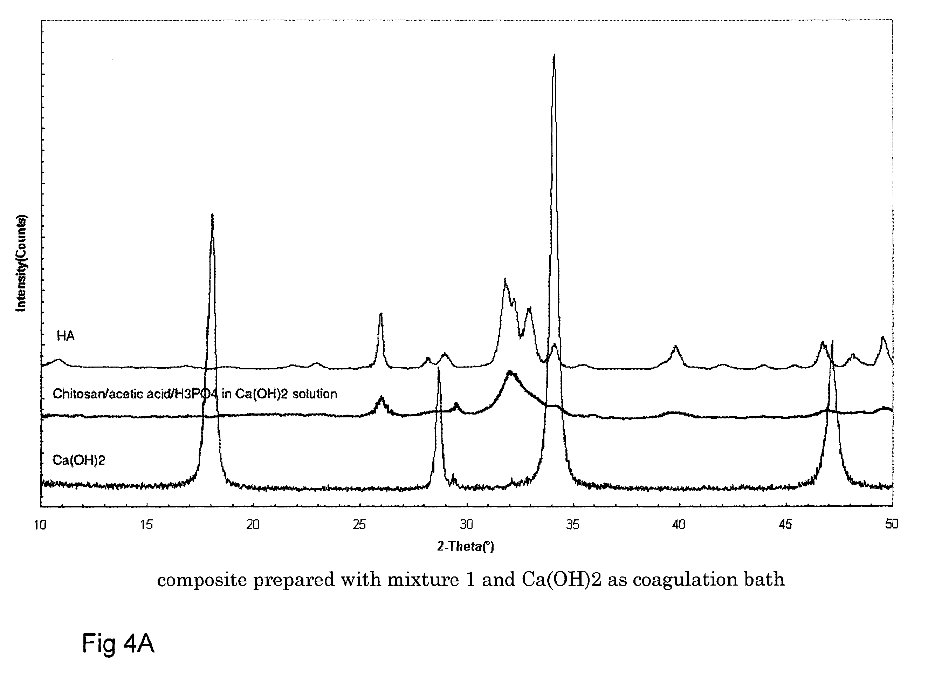

The scaffold (5 layers) prepared with processing mixture 1 injected into a 0.5 M Ca(OH)₂ coagulation bath showed a white inhomogeneous surface. The formation of hydroxyapatite was apparent to be restricted to the surface area of the prepared layer. The dried samples were analysed by XRD about the formation of hydroxyapatite (see fig. 4a) and compared with graphs of Ca(OH)₂ as well as with pure hydroxyapatite powders..

The X-ray graph of the scaffold sample clearly shows the existence of hydroxyapatite. An analysis of the centre of the sample after cutting off the outer regions revealed only the existence of chitosan. Therefore, an anisotropic scaffold was formed with a dense HA rich surface and a chitosan centre without or with only a small amount of HA particles.

### RM processing with mixtures 2a and 2b

Different scaffolds (20 layers) were prepared with processing mixture 2a and 2b into a NaOH coagulation bath. Ca (NO₃)₂ as well as CaCl₂ were used as Ca²⁺ donor. A coagulation bath with a 0.1 M concentration of NaOH resulted in the very stable scaffold.

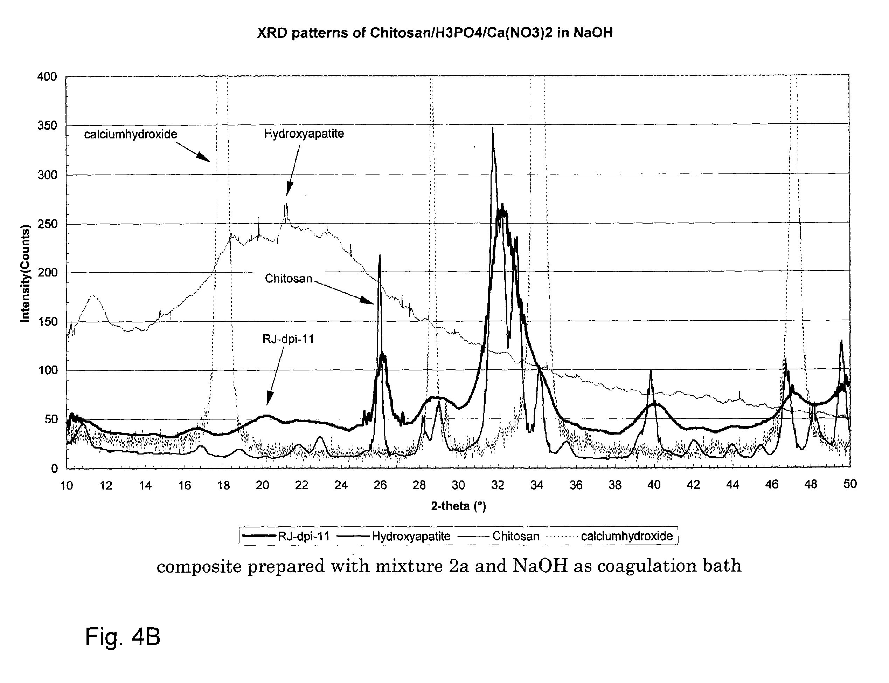

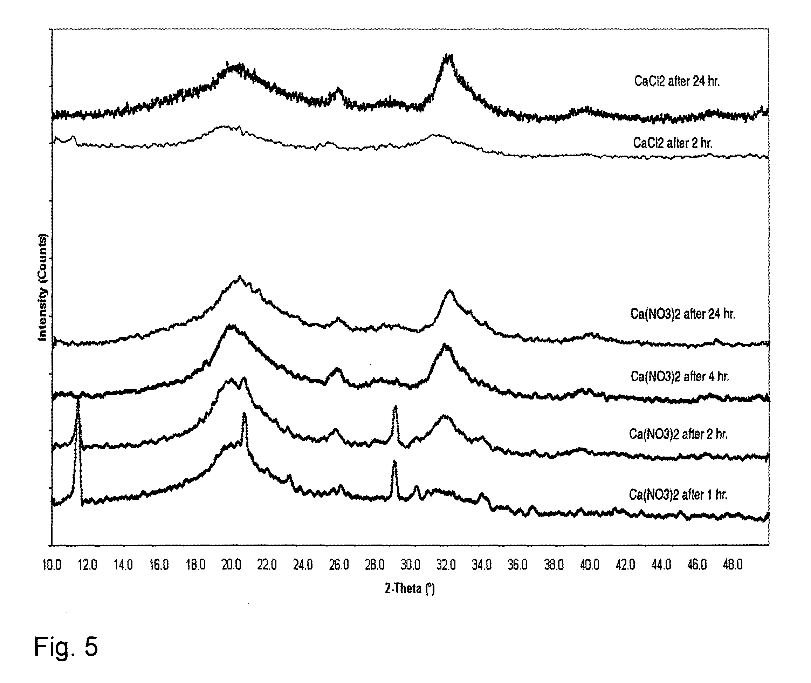

The dried samples were analysed by XRD about the formation of hydroxyapatite (see fig. 4b) and compared with graphs of chitosan, Ca(OH)₂ as well as with pure hydroxyapatite powders. The speed of formation of HA was determined using time dependent X-ray analysis. Therefore samples were prepared and analysed after different rest times in the coagulation bath. The results are shown in fig. 5 for the sample prepared with processing mixture 2a - Ca(NO₃)₂ (4 curves nearest to bottom) and processing mixture 2b - CaCl₂ (2 curves at the top).

Scaffolds with different chitosan : HA ratios (see table 1) of 20 layers were printed using the mixture 2b and stayed overnight in the coagulation bath. The produced scaffold showed a good dimensional stability, good adhesion of the single layers and perfect homogeneity.

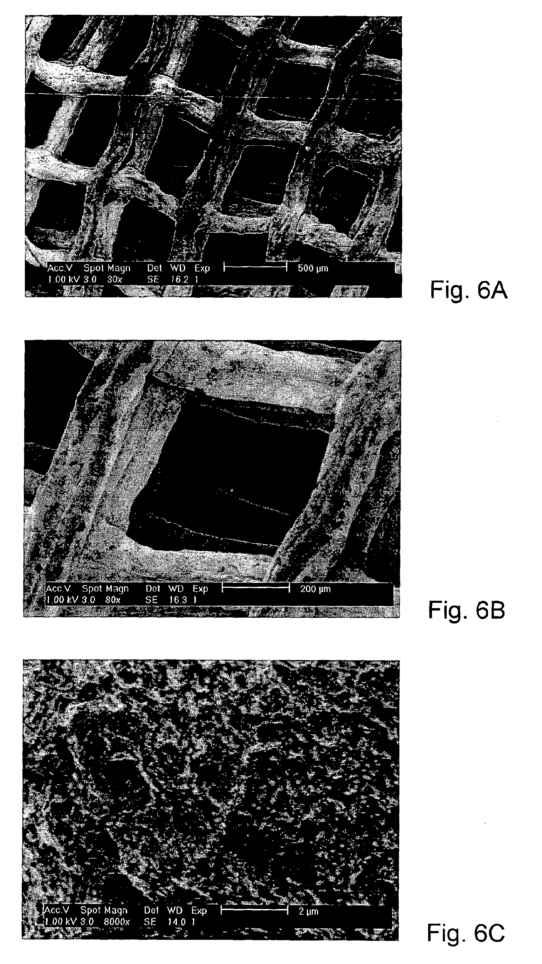

The morphology of the dried scaffold was examined with a Scanning Electron Microscope (ESEM) (see fig.6). The material was homogeneous and no agglomeration of HA or phase separation was found on the surface (see fig. 6a and b) or at the inside (see fig. 6c) of the scaffold. The pore size of the scaffold after drying was around 500 µm which is an advantageous size for the growth and attachment of cells.

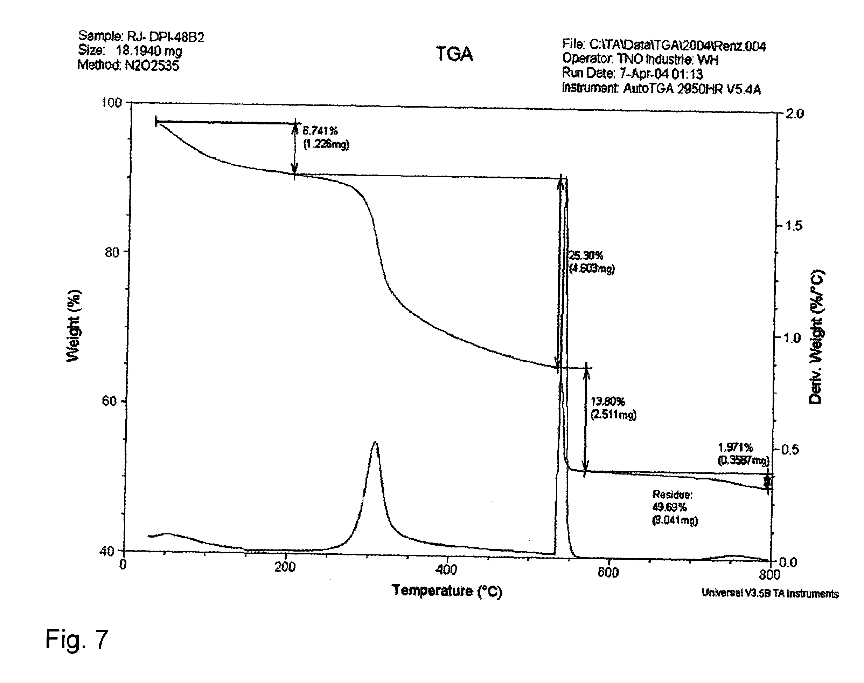

Other experiments were carried out to determine the highest possible ratios HA : chitosan to vary the mechanics of the final product in a wider range and to prepare a highly filled polymer scaffold. Different ratios of HA and chitosan (see table 1) were prepared by using processing mixture 2b and examined with TGA measurements (see figure 7).

The calculated (theoretical) amount of HA differed from the found HA amount in the prepared final scaffolds (see table 2).

**Table 2: TGA results of different scaffolds based on processing mixture 2b**

| **Calculated ratio w/w% of chitosan : HA** | **Found ratio w/w% of chitosan : HA (TGA measurements)** |
|---|---|
| 60 : 40 | 72.6 : 27.4 |
| 50 : 50 | 61.5 : 38.5 |
| 40 : 60 | 55.7 : 44.3 |
| 30 : 70 | 45.2 : 54.8 |

Depending on the starting mixture and/or the period during which the article is kept in the coagulation bath, isotropic or anisotropic scaffolds can be prepared via a 3D plotting process. A variation of properties can be achieved by changing the chitosan : HA ratio. The invention offers a highly flexible and fast way of manufacturing of shaped articles, such as biologically active implants of a specific shape, on demand.

## Claims

1. Method for manufacturing a composite article,
- providing a fluid mixture comprising (i) a polymeric matrix material and (ii) at least one dissolved inorganic component;
- forming the fluid mixture into a desired shape by Rapid Manufacturing (RM);
- allowing the matrix material to solidify;
- and forming solid particles comprising the inorganic component in the matrix material.

2. Method according to claim 1, wherein the solidification of the matrix material is effected by at least one of the following measures: subjecting the matrix material to a temperature at which the matrix material solidifies; exposing the matrix material to radiation; contacting the matrix with a liquid having a pH at which the matrix material solidifies; contacting the matrix material with a liquid having an ionic strength at which the matrix material solidifies; and contacting the matrix material with a non-solvent for the matrix material.

3. Method according to any one of the preceding claims, wherein the fluid mixture is shaped and the matrix material is allowed to solidify by contacting the shaped matrix material with a coagulation medium, preferably in a coagulation bath.

4. Method according to any one of the preceding claims, wherein the particle formation is carried out in a coagulation bath or by spraying the surface shaped matrix material with a coagulation medium and thus allowing the necessary components for the formation of the particles to migrate from the coagulation bath into the article.

5. Method according to claim 3 or 4, wherein the shaped mixture is kept in the coagulation bath for a period of at least 0.5 hour, preferably at least 3 hours, more preferably 8 to 48 hours.

6. Method according to any one of the claims 3-5, wherein the coagulation medium comprises an alkaline liquid.

7. Method according to any one of the preceding claims, wherein the matrix material is selected from the group consisting of (hetero)polysaccharides, (poly)peptides and polyesters and mixtures thereof, preferably from the group consisting of chitosan, alginate, carrageenan, pectin, agar, xanthan and starch.

8. Method according to any one of the preceding claims, wherein the one or more inorganic components are selected from the group consisting of inorganic anions and inorganic cations.

9. Method according to claim 8, wherein one or more inorganic anions are selected from the group consisting of phosphates, carbonates, sulphides, hydroxides and combinations thereof.

10. Method according to claim 8 or 9, wherein one or more inorganic cations are selected from the group consisting of metal ions, preferably from calcium, magnesium and iron.

11. Method according to any one of the preceding claims, wherein the amount of the matrix material is in the range of 20 to 99 wt. % based on the total weight of the matrix material and inorganic component and the amount of the at least one inorganic component is in the range of 1 to 80 wt. % based on the total weight of the matrix material and inorganic component.

12. Method according to any one of the preceding claims, wherein the article is selected from the group consisting of implants, foams and membranes.

13. Method according to any one of the preceding claims, wherein the formed solid particles are nanoparticles.

14. Shaped article, obtainable by a method according to any one of the preceding claims.

15. Shaped article, according to claim 14, wherein the article is either isotropic or anisotropic.

16. Shaped article, according to claim 14 or 15, wherein the particles in the article are essentially unagglomerated.
